Europäisches Patentamt

European Patent Office

Office européen des brevets

(19)

(11) Publication number: **0 183 289**

A1

(12) EUROPEAN PATENT APPLICATION

(21) Application number: **85201724.3**

(22) Date of filing: **22.10.85**

(51) Int. Cl.⁴: **C 07 D 239/26
B 01 J 23/74**

(30) Priority: **26.10.84 NL 8403259**

(43) Date of publication of application:
**04.06.86 Bulletin 86/23**

(84) Designated Contracting States:
**AT BE CH DE FR GB IT LI NL SE**

(71) Applicant: **STAMICARBON B.V.
Mijnweg 1
NL-6167 AC Geleen(NL)**

(72) Inventor: **van der Stoel, Roland Emile
Schout Boutenstraat 27
NL-6121 HC Buchten(NL)**

(74) Representative: **Leherte, Georges Maurice Lucien
Marie et al,
Octrooibureau DSM P.O.Box 9
NL-6160 MA Geleen(NL)**

(54) Process for preparing pyrimidine.

(57) Process for preparing pyrimidine in the gas phase, characterized in that 2-aminopyrimidine is contacted with a nickel and/or cobalt catalyst in the presence of water at a temperature of 200–550°C and subsequently pyrimidine is recovered from the resulting reaction mixture.

JAS/WCH/JB/WP/ag

STAMICARBON B.V. (Licensing subsidiary of DSM)

## PROCESS FOR PREPARING PYRIMIDINE

The invention relates to a process for preparing pyrimidine in the gas phase. Pyrimidine is used, inter alia, as intermediate in the synthesis of organic compounds such as, for instance, crop protection chemicals.

A process as described in the opening lines is known from Yakugaku Zasshi 96: 1005-1012 (1976). This article relates to the gas phase synthesis of 2-alkylpyrimidines from diaminopropane and aldehydes in the presence of four catalysts, viz. Pd (2 %)-$Al_2O_3$, Pt (2 %)-$Al_2O_3$, Rh (2 %)-$Al_2O_3$ and Pt (1 %)-Rh (1 %)-$Al_2O_3$. In the various cyclization experiments the best results were achieved according to this article with the Pt-Rh catalyst and with the Rh catalyst. The Pd catalyst was hardly active. Incidentally the article mentions a pyrimidine yield from diaminopropane and formaldehyde of 6 % (in addition to 6 % 2-methylpyrimidine and 19 % 2-ethylpyrimidine). From table I it can be concluded that in this experiment the Pt-Rh catalyst was used. An explanation for the low yield of pyrimidine was sought by the authors at the end of their article in the instability of the proposed intermediate hexahydropyrimidine, as well as in the susceptibility of this intermediate to side reactions.

The same research group describes in Yakugaku Zasshi 97: 373-381 (1977) the reaction of diaminopropane with methanol to form pyrimidine with a yield of 7 %. Details of this reaction are not described, but here again the catalyst used was probably Pt (1 %)-Rh (1 %)-$Al_2O_3$.

The invention now provides a process for preparing pyrimidine with a higher, economically interesting yield. The process according to the invention for preparing pyrimidine in the gas phase is charac-

0183289

terized in that 2-aminopyrimidine is contacted with a nickel and/or cobalt catalyst in the presence of water at a temperature of 200-550 °C and subsequently pyrimidine is recovered from the resulting reaction mixture. This makes it possible for a good yield of pyrimidine to be obtained in one reaction step. A synthesis for preparing 2-aminopyrimidine by a reaction of tetramethoxypropane with guanidine is described, for instance, in Aust. J. Chem. 20 (1967), pages 1595-1600. The starting compound 2-aminopyrimidine is commercially available.

In the process according to the invention the amount of water used can be varied, for instance between 1-100 moles per mole starting compound, but preference is given to an amount of 5-50 moles per mole starting compound.

The temperature at which 2-aminopyrimidine is contacted with the catalyst is preferably 250-400 °C.

The said preferred ranges in so far as the amount of water per mole staring compound and the temperature are concerned are preferred, because under these conditions the selectivity to pyrimidine is the highest.

In the process according to the invention a nickel and/or cobalt catalyst is used, which catalyst is applied to a carrier, if so desired. The amount of nickel and/or cobalt as a percentage of the total catalyst may vary within wide limits, for instance between 1 and 100 %. Suitable carriers are, for instance, aluminium oxide, silicon oxide-aluminium oxide, silicon oxide and magnesium oxide. Such catalysts, whether or not on a carrier, are commercially available.

The process according to the invention can be carried out while using an inert diluent such as, for instance, nitrogen. A regular evaporation of liquid starting compound can then be achieved. To this end hydrogen can also be used.At the beginning of the reaction the use of hydrogen has the extra advantage of the catalyst being activated.

For the actual realization of the process according to the invention the modes of realizing gas phase reactions known per se are eligible, for instance the mode of realization in which the gaseous starting mixture is passed over a fixed-bed or over a so-called fluid-bed catalyst. The space velocity of the starting mixture brought into the gas phase may be varied, for instance between 100 and 10.000 ml starting compound per millilitre catalyst material (bulk density) per hour. The pressure at which the reaction in the gas phase takes place is not important as such, so that the reaction will generally be carried out at autogenous pressure.

The further processing of the pyrimidine obtained in the reaction can be effected in a manner known per se by cooling and subsequently, for instance, distillation and extraction.

The invention is further elucidated in the following examples.

Example I

To a vertical tubular reactor with a diameter of 17 mm and a length of 400 mm, containing at the bottom a zone of 10 ml catalyst, bounded at its top by a layer of inert ceramic material and provided with a heating jacket, a solution of 22 %-(wt) 2-aminopyrimidine in water was metered. Also, at first hydrogen was passed through and afterwards nitrogen.

The catalyst used consisted of nickel on an aluminium oxide carrier (60 % (wt) nickel).

In an evaporator the liquid starting mixture was heated to 250 °C to bring this mixture into the gas phase. Subsequently the temperature in the column was raised as shown in the table. After operating periods as mentioned in the table the reaction gas was cooled for 1 hour to 12 °C. The composition of the product condensed in this cooling was determined gaschromatographically. On the basis of this determination, as well as of the weight of the amount of 2-aminopyrimidine passed over in the relative period of 1 hour, the conversion of 2-aminopyrimidine and the selectivity to pyrimidine could be calculated. The results obtained were as follows.

Table I

| operating period in hours | 8 | 23 | 26 | 31 | 34 |
|---|---|---|---|---|---|
| GHSV (ml gas/ml catalyst.$h^{-1}$) | 2400 | 2400 | 2600 | 1900 | 2100 |
| temperature in °C | 310 | 310 | 340 | 310 | 340 |
| molar ratio $H_2$/2-aminopyrimidine | 8 | 8 | 8 | - | - |
| molar ratio $N_2$/2-aminopyrimidine | - | - | - | 0,4 | 1 |
| conversion of 2-aminopyrimidine | 58 | 24 | 30 | 22 | 50 |
| selectivity to pyrimidine | 35 | 43 | 58 | 72 | 63 |

Example II

In the manner described in Example I 2-aminopyrimidine was deaminated while applying, however, a cobalt catalyst on an aluminium oxide carrier (35 % (wt) cobalt). The results obtained were as follows.

Tabel II

| operating period in hours | 8 | 16 |
|---|---|---|
| GHSV (ml gas/ml catalyst.$h^{-1}$) | 2400 | 2400 |
| temperature in °C | 310 | 310 |
| molar ratio $H_2$/2-aminopyrimidine | 8 | 8 |
| molar ratio $N_2$/2-aminopyrimidine | - | - |
| conversion of 2-aminopyrimidine | 70 | 40 |
| selectivity to pyrimidine | 22 | 28 |

## CLAIMS

1. Process for preparing pyrimidine in the gas phase, characterized in that 2-aminopyrimidine is contacted with a nickel and/or cobalt catalyst in the presence of water at a temperature of 200-550 °C and subsequently pyrimidine is recovered from the resulting reaction mixture.

2. Process according to claim 1, characterized in that per mole 2-aminopyrimidine 5-50 moles water is used.

3. Process according to claim 1 or 2, characterized in that the contacting with the catalyst takes place at a temperature of 250-400 °C.

4. Process according to any one of claims 1-3, characterized in that during the reaction hydrogen is supplied.

5. Pyrimidine obtained while applying the process according to any one of the preceding claims.

European Patent
Office

**EUROPEAN SEARCH REPORT**

0183289

Application number

EP 85 20 1724

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| A | JOURNAL OF THE CHEMICAL SOCIETY, (PERKIN I), no. 11, 1980, pages 2554-2560, London, GB; A. KATRITZKY et al.: "Reductive deamination of primary amines" * Page 2554,2559,2560 * | 1 | C 07 D 239/26 B 01 J 23/74 |

TECHNICAL FIELDS
SEARCHED (Int Cl 4)

C 07 D 239/00

The present search report has been drawn up for all claims

| Place of search | Date of completion of the search | Examiner |
|---|---|---|
| THE HAGUE | 31-01-1986 | FRANCOIS J.C.L. |